# EUROPÄISCHE PATENTSCHRIFT

(11) **EP 2 635 318 B1**
(45) Veröffentlichungstag und Bekanntmachung des Hinweises auf die Patenterteilung: **15.07.2015**
(21) Anmeldenummer: 11782525.7
(22) Anmeldetag: 04.11.2011
(51) Int. Cl.: A61M 1/16

(54) **HALTEVORRICHTUNG FÜR EINE MOBILE HERZ-LUNGEN-MASCHINE**
HOLDING DEVICE FOR A MOBILE HEART-LUNG MACHINE
DISPOSITIF DE RETENUE POUR UNE MACHINE COEUR-POUMON MOBILE

(30) Priorität: 04.11.2010 CH 18512010
(43) Veröffentlichungstag der Anmeldung: 11.09.2013
(73) Patentinhaber: Born, Frank, 8268 Salenstein (CH)
(72) Erfinder: Born, Frank, 8268 Salenstein (CH)
(74) Vertreter: Hasler, Erich
(86) Internationale Anmeldenummer: PCT/CH2011/000265
(87) Internationale Veröffentlichungsnummer: WO 2012/058778

(56) Entgegenhaltungen:
- EP-A2- 1 625 862
- DE-A1- 4 343 334
- DE-A1- 19 702 098
- DE-B3-102008 026 989

## Beschreibung

Die vorliegende Erfindung betrifft eine Haltevorrichtung für eine mobile Herz-Lungen-Maschine mit einem Rahmen und daran angeordneten Mitteln zur Befestigung, insbesondere zur Verwendung in Fahrzeugen oder Fluggeräten.

Medizinische Perfusionssysteme, welche heutzutage zur Kreislaufstabilisierung eingesetzt werden, sind in fast allen städtischen Krankenhäusern mit einem hohen Grad an Versorgungsleistung vorhanden. Im Unterschied zu diesen sind solche medizinischen Systeme in ländlichen Spitälern gewöhnlich nicht vorhanden. Um Patienten, welche an einem Herzkreislaufversagen leiden, eine frühe angemessene Behandlung anbieten zu können, werden miniaturisierte, transportable Herz-Lungen Hilfssysteme benötigt.

Ein wichtiger Aspekt der Patientenversorgung betrifft beispielsweise den Transport zwischen Spitälern, d.h. der Transport von Spitälern mit einem niedrigen Versorgungsgrad zu einer Klinik mit einem sehr hohen Versorgungsgrad. Auf der einen Seite ist ein solcher Transport oftmals eine nötige Voraussetzung für das Überleben des Patienten. Auf der anderen Seite stellt ein solcher Transport jedoch ein hohes Risiko für den Patienten dar. Um die Risiken für Patienten mit einem instabilen Kreislauf zu minimieren, sind extrakorporale Perfusionssysteme während der Transportphase eine Option.

Bisher eingesetzte Perfusionssysteme sind nicht für längere Transportzeiten ausgelegt. Sie sind in der Regel zu gross und unhandlich, sodass deren Einsatz bei Patiententransporten umständlich und aufwändig ist. Auch können sie beim Einsatz in Fahrzeugen und Flugzeugen ein Sicherheitsrisiko darstellen, wenn es zu starken positiven oder negativen Beschleunigungen kommt. Unter solchen Bedingungen besteht die Gefahr, dass die für die Notversorgung eingesetzten Geräte sich losreissen und den Patienten oder Begleitpersonen verletzen können.

Ein solches mobiles System ist in der Patentschrift DE 43 43 334 offenbart.

Aufgabe der vorliegenden Erfindung ist es, eine Haltevorrichtung für eine mobile Herz-Lungen-Maschine vorzuschlagen, die die vorerwähnten Probleme vermeiden hilft. Es ist daher insbesondere ein Ziel, eine Haltevorrichtung vorzuschlagen, die für den Einsatz in beliebigen Fahr- und Flugzeugen geeignet ist. Ein weiteres Ziel ist es, ein Notfallversorgungssystem vorzuschlagen, welches kostengünstig ist und auch die in der Luftfahrt geltenden, strengen Sicherheitsvorschriften erfüllt.

Diese Aufgabe wird durch den Gegenstand des unabhängigen Anspruchs 1 gelöst. Vorteilhafte Weiterbildungen der Erfindung gehen aus den Merkmalen der abhängigen Ansprüche hervor.

Erfindungsgemäss wird die Aufgabe bei einer Haltevorrichtung gemäss Oberbegriff von Anspruch 1 dadurch gelöst, dass eine Grundplatte vorgesehen ist, an welcher eine Mehrzahl von Halterungen für die austauschbare Befestigung von Komponenten einer mobilen Herz-Lungen-Maschine befestigt sind. An der Grundplatte sind zwei in Abstand voneinander vorgesehene Schlitze vorgesehen, um die Grundplatte mittels Fittingen am Boden eines Flugzeuges fixieren zu können. An der Grundplatte sind wenigstens eine erste Halterung zur lösbaren Befestigung einer Gasflasche, eine zweite Halterung zur lösbaren Befestigung einer Antriebseinheit, und eine dritte Halterung zur Befestigung eines Oxygenators vorgesehen. Die erfindungsgemässe Haltevorrichtung hat den Vorteil, dass die nötigen Komponenten für die Notfallversorgung eines an Herz -Kreislaufproblemen leidenden Patienten auf kleinstem Raum an der Haltevorrichtung montier- und austauschbar sind. Durch das Vorsehen von zwei Schlitzen entsprechender Länge kann die Haltevorrichtung in allen gängigen Helikoptern und Flugzeugen fixiert werden. Die mit entsprechenden Gerätschaften und Versorgungseinheiten ausgerüstete Haltevorrichtung ermöglicht daher den sicheren Transport eines schwer kranken Patienten von einem Spital mit einem niedrigen Versorgungsgrad zu einem solchen mit einem hohen Versorgungsgrad. Eine Besonderheit der erfindungsgemässen Vorrichtung ist, dass diese sowohl Herz- als auch Lungen- und Nierenunterstützung bieten kann.

Vorteilhaft sind die erste und die zweite Halterung näher zur Grundplatte angeordnet als die dritte Halterung. Das heisst, die schwereren Komponenten sind vorzugsweise näher zur Grundplatte als die leichteren Komponenten. Dies hat den Vorteil, dass die Halterungen weniger stark ausgelegt sein müssen.

Gemäss einer bevorzugten Ausführungsform ist an der Grundplatte eine dritte Halterung zur lösbaren Befestigung einer venösen Blasenfalle vorgesehen. Durch die Blasenfalle kann sichergestellt werden, dass das dem Patienten zugeführte Blut keine Luftbläschen enthält.

Zweckmässigerweise ist die Vorrichtung aus einem Leichtmetall, insbesondere Aluminium oder Titan, hergestellt. Dies ist von Bedeutung, da die mobile Haltevorrichtung möglichst leichtgewichtig sein sollte. Vorliegend wiegt eine voll bestückte Haltevorrichtung weniger als 20 Kilo und vorzugsweise weniger als 16 Kilo.

Vorteilhaft ist an der Grundplatte ein Bügel angeordnet, welcher als Haltegriff dienen kann. Dieser ist vorzugsweise ungefähr oberhalb des Schwerpunktes angeordnet, sodass die Haltevorrichtung beim Tragen nicht kippt und der Träger in einer komfortablen, aufrechten Position laufen kann. An einer Seitenkante der Grundplatte können zusätzlich zwei in Abstand voneinander angeordnete Klemmelemente vorgesehen sein. Diese ermöglichen die Befestigung der Haltevorrichtung in einer ungefähr vertikalen Position an Befestigungsschienen, welche beispielsweise an Krankenbetten vorgesehen sind.

Eine bevorzugte Ausführungsform sieht vor, dass an der Grundplatte eine vierte Halterung zur lösbaren Befestigung eines Hämo/Dialysefilters vorgesehen ist. Durch den Einsatz eines Hämofilters können auch Patienten versorgt werden, die an einer Niereninsuffizienz leiden. In diesem Ausrüstungszustand kann die bestückte Haltevorrichtung sowohl Herz- als auch Lungen- und Nierenunterstützung bietet.

Die vierte Halterung für den Oxygenator kann an der ersten Halterung befestigt sein, wobei jedoch die vierte Halterung quer zur ersten Halterung angeordnet ist.

Die erste Halterung besitzt vorzugsweise wenigstens zwei in Abstand voneinander angeordnete Befestigungsringe, wobei wenigstens einer der Befestigungsringe als Klemmring ausgebildet ist, um die Gasflasche festklemmen zu können. Die Befestigungsringe fluchten miteinander, sodass diese eine zylindrische Gasflasche umgreifen und fixieren können. Eine Stoppvorrichtung in axialem Abstand der Befestigungsringe dient als Anschlag, um sicherzustellen, dass die Gasflasche bei einem Unfall nicht aus der Halterung rutschen kann.

Vorteilhaft ist an der Grundplatte ein Adapter zur lösbaren Befestigung einer venösen Blasenfalle vorgesehen. Durch die Blasenfalle kann sichergestellt werden, dass das dem Patienten zugeführte Blut keine Luftbläschen enthält. Die Blasenfalle ist vorteilhaft, beispielsweise mittels eines Bajonettverschlusses, an den Adapter ankoppelbar.Dies ermöglicht ein sehr rasches Umsetzen der Blasenfalle. Vorzugsweise sind zwei Adapter vorgesehen, wobei der eine Adapter relativ zum anderen Adapter um 90° verdreht angeordnet ist, sodass die Blasenfalle - unabhängig davon, ob die Haltevorrichtung horizontal am Boden oder vertikal an einem Krankenbett oder einer Krankenbahre befestigt ist - in der gewünschten aufrechten Position montierbar ist. Gemäss einer bevorzugten Ausführungsform ist am Kopf des Oxygenators derselbe Adapter ausgebildet oder vorgesehen wie an der Grundplatte. Dies erlaubt eine platzsparende Blasenfalle, wenn die Grundplatte der Haltevorrichtung in der senkrechten Position ist.

Eine zweckmässige Ausführungsform sieht vor, dass die Grundplatte eine ungefähr rechteckige Grundform hat und die Schlitze in Längsrichtung der Grundplatte verlaufen. Die Grundplatte und die Halterungen sind vorteilhaft aus einem Leichtmetall oder faserverstärktem Kunststoff hergestellt.

Durch das Vorsehen eines Supports an einem Ende der Haltevorrichtung kann eine Steuer- und Überwachungsvorrichtung direkt an der Haltevorrichtung befestigt werden. Dies hat den Vorteil, dass alle wichtigen Komponenten auf kleinstem Raum angeordnet sind.

Die Erfindung wird nachfolgend anhand der Figuren näher im Detail erläutert. Es zeigt:

| | |
|---|---|
| Fig. 1 | eine erste perspektivische Ansicht eines Ausführungsbeispiels einer Haltevorrichtung mit mehreren offenen Halterungen für die Komponenten eines Notfallversorgungssystems für Patienten, deren Herz-Kreislaufsystem stabilisiert werden muss; |
| Fig. 2 | eine zweite perspektivische Ansicht der Haltevorrichtung von der gegenüberliegenden Seite; |
| Fig. 3 | eine dritte perspektivische Ansicht der Haltevorrichtung wie in Fig. 2, wobei die Halterungen jedoch geschlossen sind; |
| Fig. 4 | eine perspektivische Ansicht der Haltevorrichtung mit den entsprechenden Geräten von oben; |
| Fig. 5 | eine perspektivische Ansicht der Haltevorrichtung von Fig. 4 von unten. |

Die in den Figuren gezeigte Haltevorrichtung 11 für ein Perfusionssystem 13 besitzt eine Grundplatte 15, an welcher wenigstens eine erste Halterung 17 für die lösbare Befestigung einer Gasflasche 19, eine zweite Halterung 21 für die lösbare Befestigung einer Antriebseinheit 23 und eine dritte Halterung 25 für die lösbare Befestigung eines Oxygenators 27 angeordnet sind. Danebst kann noch ein Adapter 29 für die Aufnahme einer venösen Blasenfalle 31 und eine vierte Halterung 33 für einen Hämofilter 35 vorgesehen sein. Die einzelnen Halterungen 17,21,25 und 33 und der Adapter 29 sind so bemessen, dass im Handel erhältliche Geräte und Gasflaschen eingesetzt und montiert werden können. Ist die Haltevorrichtung 11 bestückt, können Patienten mit Herz-Kreislaufproblemen über mehrere Stunden voll versorgt werden, sodass auch ein mehrstündiger Transport von einem Spital mit einem niedrigen Versorgungsgrad zu einem solchen mit einem hohen Versorgungsgrad erfolgen kann.

Die Haltevorrichtung für eine Notfallversorgungseinrichtung ist so ausgelegt, dass sie in gängigen Fahrzeugen und Flugzeugtypen, einschliesslich von Rettungshelikoptern, sicher montiert werden kann, sodass auch bei sehr grossen Beschleunigungen kein Sicherheitsrisiko besteht. So ist die Haltevorrichtung vorzugsweise aus Leichtmetall, z.B. einer Aluminium-Legierung, hergestellt. Die Haltevorrichtung und die erwähnten Halterungen sind so ausgelegt, dass diese mindestens folgenden Belastungen standhalten können:

| Richtung der Belastung | Belastungsfaktor in g (Erdbeschleunigung) |
|---|---|
| Aufwärts | 4 g |
| Nach vorne | 16 g |
| Seitwärts | 8 g |
| Abwärts | 20 g |
| Rückwärts | 1.5 g |

Die eingesetzte Aluminium- Legierung entspricht einer solchen, wie sie von der Luft- und Raumfahrtbehörde (ESA) zugelassen ist, sodass die Haltevorrichtung die für Flugzeuge geltenden Sicherheitsvorschriften erfüllt. Grundsätzlich können anstelle von Aluminium auch andere Leichtmetalle wie Titan oder Magnesium oder deren Legierungen verwendet werden.

Damit die Haltevorrichtung in allen gängigen Flugzeugtypen befestigt werden kann, sind in der Grundplatte 15 zwei voneinander beabstandete, parallele Schlitze 37 vorgesehen. Die Länge der beiden Schlitze 37 ist so gewählt, dass die Haltevorrichtung an den in den verschiedenen Fluggeräten standardmässig am Boden vorhandenen Befestigungsschienen montiert werden kann. Da der Abstand der Befestigungsschienen in Flugzeugen zwischen 22 und 46 cm variieren kann, besitzen die Schlitze 37 vorzugsweise eine Mindestlänge von 46 cm. Dies ermöglicht die sekundenschnelle Befestigung der Haltevorrichtung in verschiedenen Flugzeugtypen und Helikoptern mittels bekannter Schnellverbinder (Fittingen), erhältlich beispielsweise von der Firma Allsafe Jungfalk, Engen, Deutschland.

Eine weitere Befestigungsmöglichkeit ist durch zwei Klemmelemente 39 gegeben, welche an einer Längsseite 41 der Grundplatte 15 vorgesehen sind. Die Klemmelemente 39 ermöglichen die Befestigung der Haltevorrichtung an üblichen T-Schienen, wie sie an Krankenbetten normalerweise vorhanden sind. Es ist jedoch denkbar, dass die Klemmelemente eine andere Gestalt aufweisen, um an anderen Systemen befestigt werden zu können. Vorliegend besitzen die Klemmelemente 39 je eine Hinterschneidung 43, mittels welcher die Klemmelemente 39 an einer T-Schiene eingehängt und mittels einer Schraube 45 festgeklemmt werden können.

Zwischen den Klemmelementen 39 ist am Rand der Grundplatte ein Haltegriff 47 vorgesehen. Dieser ist durch einen U-förmigen Bügel gebildet, dessen Schenkel 49 mit der Stirnseite an der Grundplatte 15 festgemacht sind. Die Höhe des Bügels ist vorzugsweise so gewählt, dass beim Tragen die Senkrechte durch den Schwerpunkt im Wesentlichen parallel zur Grundplatte und durch den Punkt des Haltegriffs 47 verläuft, an dem der Haltegriff 47 festgehalten wird. Dies hat den Vorteil, dass die Haltevorrichtung sich gut tragen lässt.

An dem vorderen Ende 51 der Haltevorrichtung ist ein Support 53 bestehend aus zwei in Abstand voneinander angeordneten Bügeln 55a und 55b. Diese sind durch zwei Streben 57 miteinander verbunden. Auf dem Support 53 kann ein in den Figuren nicht dargestelltes Steuer- und Überwachungsgerät abgestellt und beispielsweise mit Hilfe eines Spanngurtes, welcher um die Streben 57 und das Gerät geführt wird, befestigt werden.

An der Stirnseite des Bügels 55a ist eine Schiene 59 angeordnet, an welcher eine Mehrzahl Fittings zwecks Aufbewahrung befestigbar sind. Dies hat den Vorteil, dass die zur Befestigung der Haltevorrichtung nötigen Fittings nicht vergessen werden können.

Die erste Halterung 17 für die Gasflasche besteht aus einem Befestigungsring 61 und einem zu diesem beabstandeten Klemmring 63. Der Befestigungsring 61 hat einen Innendurchmesser, welcher dem Aussendurchmesser einer Gasflasche 19 entspricht, sodass letztere passgenau im Befestigungsring 61 aufgenommen ist. Der Klemmring 63 hat einen im Vergleich zum Befestigungsring minimalen Innendurchmesser und kann mittels einer Schraube 65 zusammengezogen werden, sodass die Gasflasche fest gehalten ist. Damit die relativ schwere Gasflasche auch bei einer negativen Beschleunigung (=Bremsvorgang) von 16 g nicht aus der Halterung 17 rutschen kann, ist in axialem Abstand vom Klemmring 63 ein Stopp 67 vorgesehen. Der Stopp 67 steht von der Grundplatte 15 ab und dient als Anschlag für die Gasflasche 19.

Die zweite Halterung 21 für die Antriebseinheit 23 besteht vorzugsweise aus einer zweiteiligen Schelle mit einem Schnellspannverschluss 69. Dieser erlaubt das rasche Auswechseln der Antriebseinheit.

Die dritte Halterung 25 für den Oxygenator 27 ist am Befestigungsring 61 festgemacht und besteht ebenfalls aus einer Schelle mit einem Schnellspannverschluss 71. Die Schelle ist um 90 Grad verdreht auf dem Befestigungsring 61 angeordnet, sodass der Oxygenator in Bezug auf die Hauptfortbewegungsrichtung 73 formschlüssig gehalten ist. Die Haltevorrichtung ist in einem Fahr- oder Flugzeug so eingebaut, dass die Vorderseite 51 in Bewegungsrichtung nach vorne gerichtet ist.

Die vierte Halterung 33 für den Hämofilter 35 besteht aus zwei in Abstand voneinander angeordneten elastischen Klemmteilen, in welche ein handelsüblicher Hämofilter eingeschnappt werden kann. Der Hämofilter ist unterhalb des Supports angeordnet und daher - wie die Gasflasche - von äusseren Schlageinwirkungen gut geschützt.

Der Adapter 29 für die venöse Blasenfalle 31 hat einen Bajonettverschluss 75, an welchen die Blasenfalle 31 gekoppelt werden kann. Im Adapter 29 wird die Blasenfalle 31 dann montiert, wenn die Haltevorrichtung am Boden eines Fluggeräts montiert wird (horizontale Montage). Wird die Haltevorrichtung in senkrechter Stellung beispielsweise an einem Krankenbett oder einer Bahre montiert, dann kann die Blasenfalle 31 am Kopf des Oxygenators 27 montiert werden. Dieser weist am Kopf vorzugsweise denselben Bajonettverschluss auf, sodass je nach Betriebslage der Haltevorrichtung die Blasenfalle jeweils in der richtigen aufrechten Stellung montiert werden kann. Dies ist von grosser Wichtigkeit, um Luftbläschen sicher mit der Unterseite nach unten aussondern zu können.

Die Grundplatte 15 ist rechtwinklig ausgebildet mit Kantenlängen von 50 x 30 cm (Länge x Breite). Das Totalgewicht der Haltevorrichtung mit den daran befestigten Komponenten liegt unterhalb von 15 Kg bei der Verwendung einer 2 Liter Gasflasche mit einem Fülldruck von 300 bar. Ein geeigneter Membran - Oxygenator mit einer stromaufwärts liegenden zentrifugalen Blutpumpe kann eingesetzt werden. Diese Oxygenatoren sind für den Einmalgebrauch bestimmt

Eine Haltevorrichtung für eine mobile Herz-Lungen-Maschine, insbesondere zur Verwendung in Fahrzeugen oder Fluggeräten, umfasst eine Grundplatte 15, in welcher zwei in Abstand voneinander angeordnete Schlitze 37 vorgesehen sind. Diese dienen der Aufnahme von Befestigungsmitteln zur Befestigung der Haltevorrichtung an Befestigungsschienen in einem Flugzeug. An der Grundplatte 15 ist eine erste Halterung 17 zur lösbaren Befestigung einer Gasflasche 19, eine zweite Halterung 21 zur lösbaren Befestigung einer Antriebseinheit 23, und eine dritte Halterung 25 zur Befestigung eines Oxygenators vorgesehen.

### Legende

- 11: Haltevorrichtung
- 13: Perfusionssystem
- 15: Grundplatte
- 17: erste Halterung
- 19: Gasflasche
- 21: Zweite Halterung
- 23: Antriebseinheit
- 25: Dritte Halterung
- 27: Oxygenator
- 29: Adapter für venöse Blasenfalle
- 31: venöse Blasenfalle
- 33: Vierte Halterung
- 35: Hämofilter
- 37: Schlitze in der Grundplatte
- 39: Klemmelemente
- 41: Längsseite
- 43: Hinterschneidung
- 45: Schraube
- 47: Haltegriff
- 49: Schenkel des Haltegriffs
- 51: vorderes Ende der Haltevorrichtung
- 53: Support
- 55: Bügel
- 57: Streben
- 59: Schiene
- 61: Ring der ersten Halterung
- 63: Klemmring
- 65: Schraube
- 67: Stoppvorrichtung
- 69: Schnellspannverschluss
- 71: Schnellspannverschluss der 3. Halterung
- 73: Hauptfortbewegungsrichtung
- 75: Bajonettverschluss

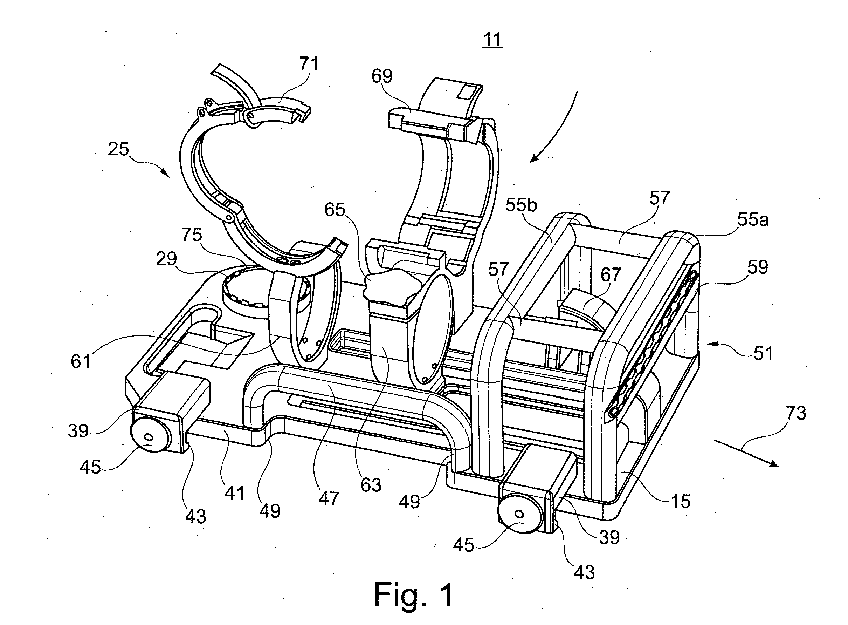

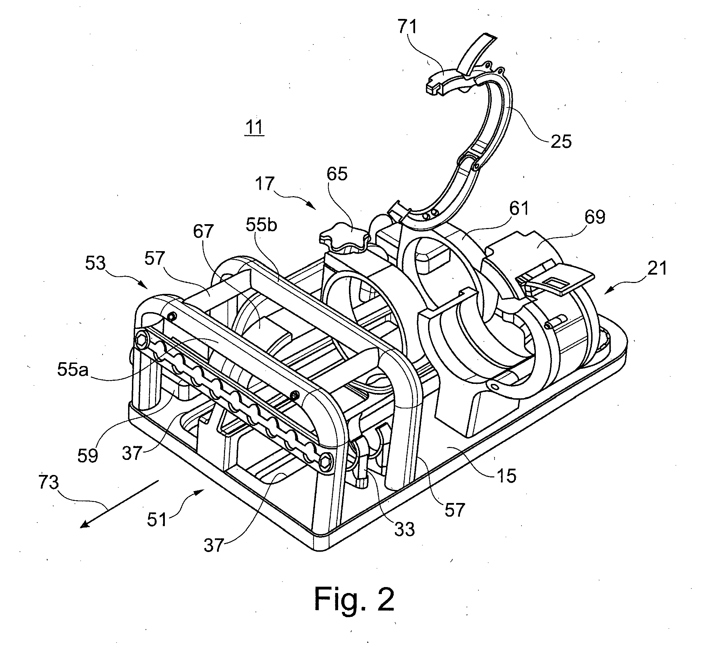

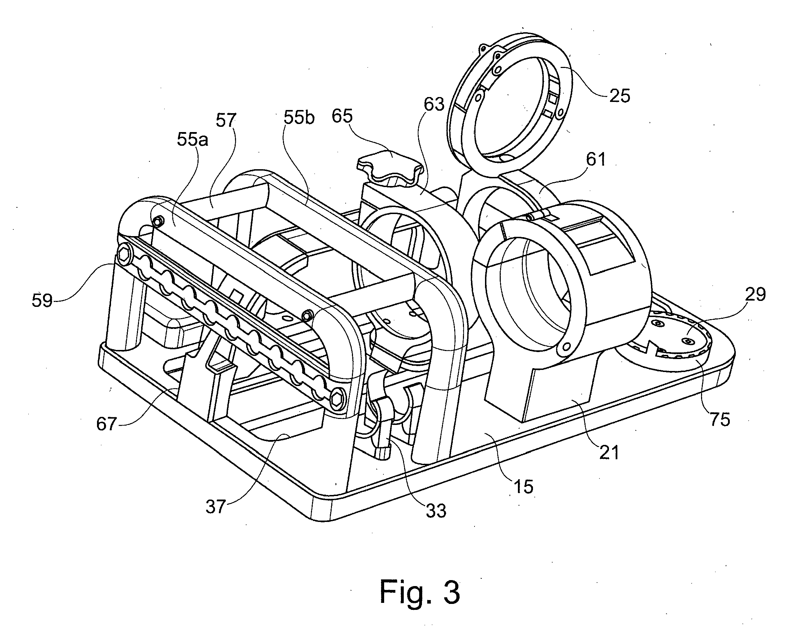

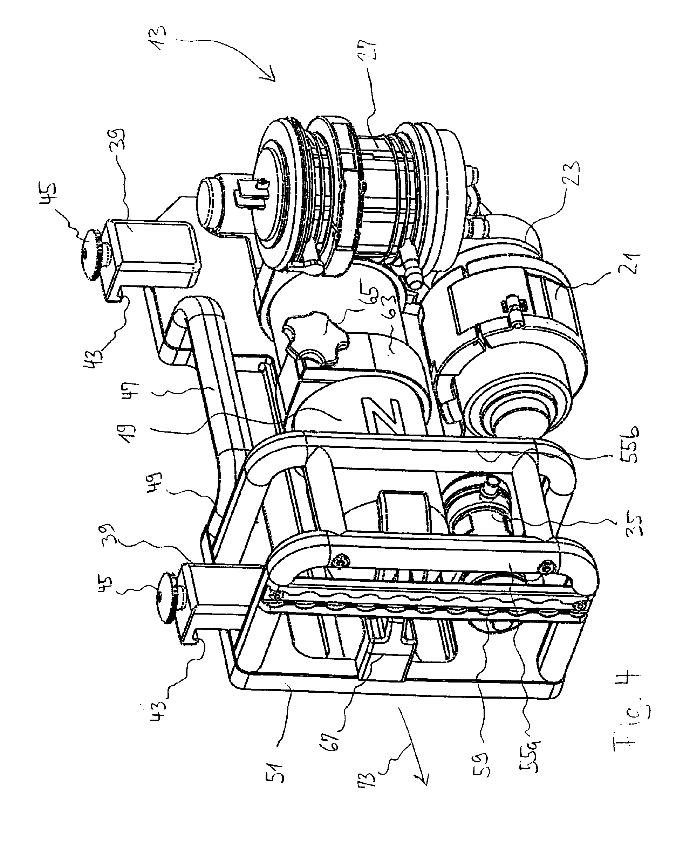

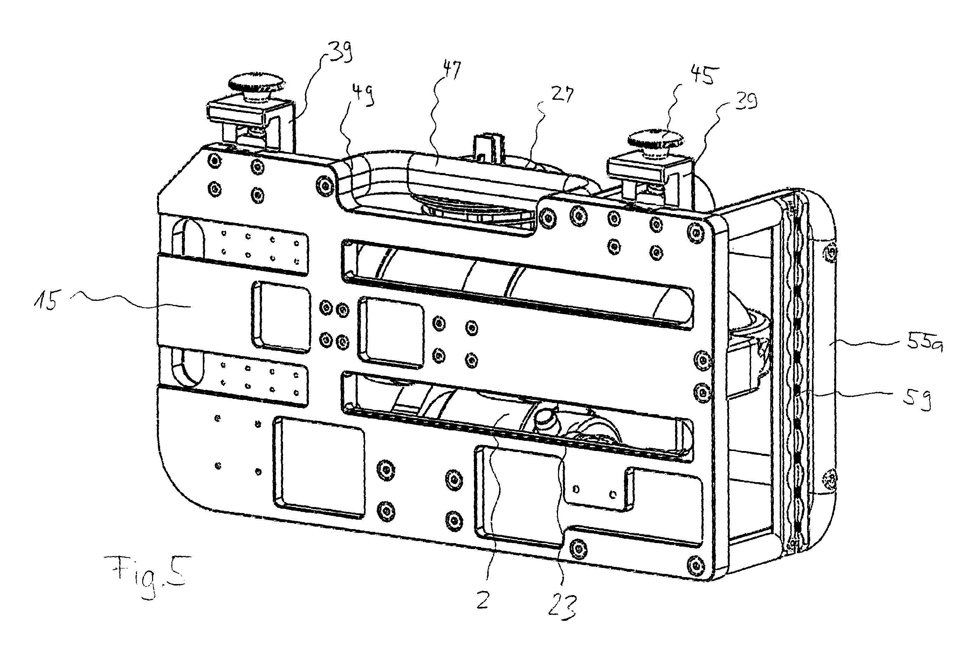

## Patentansprüche

1. Haltevorrichtung für eine mobile Herz-Lungen-Maschine mit einem Rahmen und daran angeordneten Befestigungsmitteln, insbesondere zur Verwendung in Fahrzeugen oder Fluggeräten,
einer Grundplatte (15),
einer an der Grundplatte (15) angeordneten ersten Halterung (17) zur lösbaren Befestigung einer Gasflasche (19),
einer an der Grundplatte (15) angeordneten zweiten Halterung (21) zur lösbaren Befestigung einer Antriebseinheit (23), und
einer an der Grundplatte (15) angeordneten dritten Halterung (25) zur Befestigung eines Oxygenators,
**gekennzeichnet durch**
zwei in Abstand voneinander vorgesehenen Schlitzen (37), welche in der Grundplatte (15) vorgesehen sind zur Aufnahme von Befestigungsmitteln zur Befestigung der Haltevorrichtung an Befestigungsschienen.

2. Haltevorrichtung nach Anspruch 1, **dadurch gekennzeichnet, dass** die erste und die zweite Halterung (17,21) näher zur Grundplatte (15) angeordnet sind als die dritte Halterung (25).

3. Haltevorrichtung nach Anspruch 1 oder 2, **dadurch gekennzeichnet, dass** an der Grundplatte ein Adapter für venöse Blasenfalle (29) zur lösbaren Befestigung einer venösen Blasenfalle (31) vorgesehen ist.

4. Haltevorrichtung nach einem der Ansprüche 1 bis 3, **dadurch gekennzeichnet, dass** die Haltevorrichtung aus einem Leichtmetall, insbesondere Aluminium oder Titan, hergestellt ist.

5. Haltevorrichtung nach einem der Ansprüche 1 bis 3, **dadurch gekennzeichnet, dass** an der Grundplatte (15) ein Bügel (55) angeordnet ist, welcher als Haltegriff (47) dienen kann.

6. Haltevorrichtung nach einem der Ansprüche 1 bis 5, **dadurch gekennzeichnet, dass** an einer Seitenkante der Grundplatte (15) zwei in Abstand voneinander angeordnete Klemmelemente (39) vorgesehen sind.

7. Haltevorrichtung nach einem der Ansprüche 1 bis 3, **dadurch gekennzeichnet, dass** an der Grundplatte eine vierte Halterung (33) zur lösbaren Befestigung eines Hämofilter (35) vorgesehen ist.

8. Haltevorrichtung nach einem der Ansprüche 1 bis 3, **dadurch gekennzeichnet, dass** die dritte Halterung für die Befestigung des Oxygenators an der ersten Halterung (17) angeordnet ist.

9. Haltevorrichtung nach einem der Ansprüche 1 bis 8, **dadurch gekennzeichnet, dass** an der Grundplatte ein Adapter (29) zur lösbaren Befestigung einer venösen Blasenfalle (31) vorgesehen ist.

10. Haltevorrichtung nach einem der Ansprüche 1 bis 9, **dadurch gekennzeichnet, dass** die erste Halterung durch wenigstens zwei in Abstand voneinander angeordnete Befestigungsringe (61,63) gebildet ist, wobei wenigstens einer der Befestigungsringe als Klemmring (63) ausgebildet ist.

11. Haltevorrichtung nach Anspruch 10, **dadurch gekennzeichnet, dass** die Befestigungsringe (61,63) in Längsrichtung der Grundplatte hintereinander angeordnet sind und dass im Abstand der Befestigungsringe eine Stoppvorrichtung (67) für die Gasflasche (19) vorgesehen ist.

12. Haltevorrichtung nach einem der Ansprüche 1 bis 11, **dadurch gekennzeichnet, dass** die Grundplatte eine ungefähr rechteckige Grundform hat und die Schlitze (37) in Längsrichtung der Grundplatte angeordnet sind.

13. Haltevorrichtung nach einem der Ansprüche 1 bis 12, **dadurch gekennzeichnet, dass** an einem Ende der Haltevorrichtung ein Support (53) für die lösbare Anordnung einer Steuer- und Überwachungsvorrichtung vorgesehen ist.

## Claims

1. Holding mechanism for a mobile heart-lung machine, having a frame and fastening means arranged thereon, in particular for use in vehicles or aircraft, a base plate (15), a first holding device (17), arranged on the base plate (15), for detachable fastening of a gas bottle (19), a second holding device (21), arranged on the base plate (15), for detachable fastening of a drive unit (23) and a third holding device (25), arranged on the base plate (15), for fastening an oxygenator, **characterised by** two slots (37) provided at a spacing from each other, which are provided in the base plate (15) for receiving fastening means for fastening the holding mechanism to fastening rails.

2. Holding mechanism according to claim 1, **characterised in that** the first and second holding devices (17, 21) are arranged closer to the base plate (15) than the third holding device (25).

3. Holding device according to claim 1 or 2, **characterised in that** an adapter for venous bubble traps (29) is provided on the base plate (15) for detachable fastening of a venous bubble trap (31).

4. Holding mechanism according to any one of claims 1 to 3, **characterised in that** the holding mechanism is manufactured from a light metal, in particular aluminium or titanium.

5. Holding mechanism according to any one of claims 1 to 3, **characterised in that** a bracket (55) is arranged on the base plate (15) and this can be used as a handle (47).

6. Holding mechanism according to any one of claims 1 to 5, **characterised in that** two clamping elements (39) arranged at a spacing from each other are provided on a side edge of the base plate (15).

7. Holding mechanism according to any one of claims 1 to 3, **characterised in that** a fourth holding device (33) is provided on the base plate (15) for detachable fastening of a hemofilter (35).

8. Holding mechanism according to any one of claims 1 to 3, **characterised in that** the third holding device for fastening the oxygenator is arranged on the first holding device (17).

9. Holding mechanism according to any one of claims 1 to 8, **characterised in that** an adapter (29) is provided on the base plate for detachable fastening of a venous bubble trap (31).

10. Holding mechanism according to any one of claims 1 to 9, **characterised in that** the first holding device is formed by at least two fastening rings (61, 63) arranged at a spacing from each other, wherein at least one of the fastening rings is designed as a clamping ring (63).

11. Holding mechanism according to claim 10, **characterised in that** the fastening rings (61, 63) are arranged one behind the other in the longitudinal direction of the base plate, and **in that** a stopping mechanism (67) for the gas bottle (19) is provided at a spacing from the fastening rings.

12. Holding mechanism according to any one of claims 1 to 11, **characterised in that** the base plate has a roughly rectangular basic shape and the slots (37) are arranged in the longitudinal direction of the base plate.

13. Holding mechanism according to any one of claims 1 to 12, **characterised in that** a support (53) for the detachable arrangement of a control and monitoring device is provided on one end of the holding mechanism.

## Revendications

1. Dispositif de support pour un appareil de réanimation cardio-pulmonaire mobile avec un cadre et des moyens de fixation qui y sont fixés, en particulier pour l'utilisation dans des véhicules ou des appareils volants, une plaque de base (15), un premier support (17) placé sur la plaque de base (15) pour la fixation amovible d'une bouteille de gaz (19), un second support (21) placé sur la plaque de base (15) pour la fixation amovible d'une unité d'entraînement (23) et un troisième support (25) placé sur la plaque de base (15) pour la fixation d'un oxygénateur,
**caractérisé par** deux fentes (37) prévues espacées l'une de l'autre qui sont prévues dans la plaque de base (15) pour loger des moyens de fixation pour la fixation du dispositif de support sur des rails de fixation.

2. Dispositif de support selon la revendication 1, **caractérisé en ce que** le premier et le second support (17, 21) sont placés plus près de la plaque de base (15) que le troisième support (25).

3. Dispositif de support selon la revendication 1 ou 2, **caractérisé en ce qu'**un adaptateur pour piège à bulles veineux (29) est prévu sur la plaque de base pour la fixation amovible d'un piège à bulles veineux (31).

4. Dispositif de support selon l'une des revendications 1 à 3, **caractérisé en ce que** le dispositif de support est fabriqué en un métal léger, en particulier en aluminium ou en titane.

5. Dispositif de support selon l'une des revendications 1 à 3, **caractérisé en ce qu'**un étrier (55) qui peut servir de poignée (47) est placé sur la plaque de base (15).

6. Dispositif de support selon l'une des revendications 1 à 5, **caractérisé en ce que** deux éléments de serrage (39) placés espacés l'un de l'autre sont prévus sur un bord latéral de la plaque de base (15).

7. Dispositif de support selon l'une des revendications 1 à 3, **caractérisé en ce qu'**un quatrième support (33) est prévu sur la plaque de base pour la fixation amovible d'un hémofiltre (35).

8. Dispositif de support selon l'une des revendications 1 à 3, **caractérisé en ce que** le troisième support pour la fixation de l'oxygénateur est placé sur le premier support (17).

9. Dispositif de support selon l'une des revendications 1 à 8, **caractérisé en ce qu'**un adaptateur (29) est prévu sur la plaque de base pour la fixation amovible d'un piège à bulles veineux (31).

10. Dispositif de support selon l'une des revendications 1 à 9, **caractérisé en ce que** le premier support est formé par au moins deux anneaux de fixation (61, 63) placés espacés l'un de l'autre, au moins l'un des anneaux de fixation étant configuré comme une bague de serrage (63).

11. Dispositif de support selon la revendication 10, **caractérisé en ce que** les anneaux de fixation (61, 63) sont placés l'un derrière l'autre dans le sens longitudinal de la plaque de base et qu'un dispositif d'arrêt (67) pour la bouteille de gaz (19) est prévu espacé des anneaux de fixation.

12. Dispositif de support selon l'une des revendications 1 à 11, **caractérisé en ce que** la plaque de base a une forme de base approximativement rectangulaire et que les fentes (37) sont placées dans le sens longitudinal de la plaque de base.

13. Dispositif de support selon l'une des revendications 1 à 12, **caractérisé en ce qu'**un support (53) pour le placement amovible d'un dispositif de commande et de contrôle est prévu à une extrémité du dispositif de support.
